# EUROPEAN PATENT SPECIFICATION

(11) **EP 0 437 287 B1**
(45) Date of publication and mention of the grant of the patent: **17.07.1996**
(21) Application number: 91103886.7
(22) Date of filing: 04.04.1986
(51) Int. Cl.: G01N 33/549, G01N 33/545, G01N 33/569, C12Q 1/68

(54) **A solid phase system for use in ligand-receptor assays**
Festphasen-System für Ligand-Rezeptor-Bestimmungen
Système en phase solide pour essais du type ligand-récepteur

(30) Priority: 04.04.1985 US 720036
(43) Date of publication of application: 17.07.1991
(62) Divisional of application: 86302521.9
(73) Proprietor: HYBRITECH INCORPORATED, San Diego, CA 92121 (US)
(72) Inventor: Rubenstein, Albert Samuel, Encinitas, California 92024 (US)
(74) Representative: Goldin, Douglas Michael

(56) References cited:
- EP-A- 0 119 622
- WO-A-82/02601
- WO-A-85/05451
- FR-A- 2 487 983
- GB-A- 1 420 916
- US-A- 3 951 748
- US-A- 4 338 094
- US-A- 4 444 879

## Description

This invention relates to ligand-receptor assay processes. In another aspect, it relates to a solid phase system for use in ligand-receptor assays, particularly immunoassays using monoclonal antibodies.

This application relates to the subject matter of US-A-4632901 and US-A-4727019. This application also relates to the subject matter of EP-A-253 464.

Ligand-receptor assays, particularly immunoassays, provide sensitive diagnostic tools for the in vitro detection in serum and other body fluids of analytes associated with disease and other physiological conditions of clinical significance.

In the past, immunoassays have typically relied on a polyclonal antibody preparation bound to a solid phase. In such assays, a solution of antigen labeled to permit detection is allowed to compete with antigen in a sample for the solid phase antibody. The extent to which the labeled antigen is bound to the solid phase or is detected in the liquid phase can be used as a measure of the presence and quantity of antigen in the sample being analyzed.

Subsequently, non-competitive immunometric assays became available. In these assays, a polyclonal antibody preparation bound to a solid phase is also used. The sample containing the suspected antigen is allowed to contact the solid phase in order for the antigen to bind to the antibodies on the solid phase. Typically, after an incubation step the sample is separated from the solid phase which is then washed and incubated with a solution of additional polyclonal antibodies which has been labeled, for example with a radionuclide, an enzyme, or a fluorescent moiety, to permit detection.

After this second incubation, the unbound labeled antibody is separated from the solid phase and the amount of labeled antibody in either the liquid phase or bound to the solid phase in an antibody:antigen:antibody sandwich is determined as a measure of the presence and/or concentration of antigen in the sample tested.

More recently, immunoassay processes have been modified to use monoclonal antibodies. For example, U.S. 4,376,110 describes two-site immunometric assays using pairs of monoclonal antibodies, one bound to a solid phase and the other labeled to permit detection. The use of monoclonal antibody pairs which recognize different epitopic sites on an antigen has made it possible to conduct simultaneous immunometric assays in which the antigen and labeled antibody incubations do not require the intermediate steps of prior processes.

In the foregoing immunoassay processes, the solid phase system typically comprises an antibody bound to a bead, or alternatively, an antibody coated on a material such as a membrane or filter, suitable to capture an antigen of interest. At present, the preparation of such solid phase systems characteristically requires activation procedures to facilitate the coating of an antibody to a solid support. Additionally, a backcoating procedure, involving the coating of the solid support with a substance effective to inhibit non-specific binding, is generally required. The activation and backcoating procedures are time-consuming and difficult procedures, and as a result, render the preparation of solid phase systems very costly.

In addition to the above-described limitations associated with the preparation of solid phase systems presently available, it has not been possible to assay a given sample for more than one analyte of interest simultaneously on a single solid phase system. Further, present solid phase systems lack the internal controls (i.e., positive and negative controls) which are essential for a determination of the validity and reliability of an assay. The preparation of solid phase systems for a multiple immunoassay process and/or an internal control system has been, prior to the present invention, very difficult.

EP-A-0119622 describes an integral element for a biological reaction comprising at least two layers consisting essentially of (1) a composite porous biological reaction layer comprising a fibrous material in which is dispersed a particulate material to which an active substance capable of participating in a biochemical reaction is fixed, and (2) a porous layer of a fibrous material. To prepare the two fibrous layers and to obtain the dispersion of particulate material in layer (1) a process analogous to paper making is used involving wet slurries.

US-A-4338094 describes a rigid capsule, porous to fluids (e.g. made from two die-stamped semi-spheres) in which are encapsulated particles which support antibodies.

Accordingly, there exists a need for a solid phase system which may be prepared more efficiently and which minimizes the difficulty and expense of preparation. Additionally, there exists a need for an improved solid phase system which may be utilized to assay a sample for at least two analytes of interest simultaneously. Further, there exists a need for an improved solid phase system which permits the incorporation of internal controls for the performance of assays.

The present invention provides a solid phase system for use in ligand-receptor assays, particularly immunoassays, for the detection of a selected analyte in a fluid sample. As used herein, the term "ligand-receptor assay" refers to an assay for an analyte which is detected by the formation of a complex between a ligand and another substance capable of specific interaction with the ligand, i.e. receptor. The ligand may be the analyte itself or a substance which if detected can be used to infer the presence of the analyte in a sample. Persons skilled in the art will appreciate that, depending upon the analyte of interest, a member of a specific binding pair may be either receptor or ligand depending upon assay design. In the context of the present invention, the term "ligand" encompasses antigens, haptens, antibodies, deoxyribonucleic acid (DNA), ribonucleic acid (RNA), hormones, metabolites and other naturally occurring substances of diagnostic interest having a specific binding partner therefor, i.e., the receptor of the ligand-receptor assay.

The solid phase system of the invention comprises a porous matrix, such as a membrane or filter, within which microspheres are entrapped. Preferably, the microspheres are entrapped within a discrete or defined zone of the matrix. Additionally, the microspheres, selected to have a size compatible with the pore size of the porous matrix, are bound with a receptor such as an antibody, preferably a monoclonal antibody, antigen, nucleic acid sequence or other substance capable of capturing the selected ligand when exposed to a sample containing the ligand. For example, the microspheres may be bound with an allergen capable of capturing an IgE antibody in a sample which is specific for the bound allergen.

In preferred solid phase systems, distinct groups of microspheres to which are bound an antibody, antigen or other suitable receptor substance are entrapped within discrete zones of the porous matrix so as to permit the performance of a multiple assay for the detection of at least two selected analytes. Further, distinct groups of microspheres may be entrapped within the porous matrix so as to incorporate internal control systems for the detection of selected analytes.

The present invention is also directed to an apparatus comprising, as a first member, a porous solid phase system as described above. The preferred apparatus further comprises, as a second member, an absorbent member associated with the solid phase system so as to permit the flow of a fluid sample through the solid phase system and into the second member. (The terms "solid phase system" and "first porous member" are used interchangeably herein.)

The present invention is further directed to a ligand-receptor assay process comprising, as a first step, the introduction of a fluid sample onto the solid phase system whereby, as the fluid flows through the solid phase system, the receptor bound to the microspheres captures the selected target ligand. Following the addition of the sample, a solution of a receptor conjugate capable of binding the target ligand and labeled so as to permit detection is added to the solid phase system. As used herein the term "receptor conjugate" refers to a complex comprising a receptor and a label capable of detection. In the case of an immunometric assayfor a target antigen the receptor conjugate may be labeled antibody, preferably a monoclonal antibody. Alternatively, if the target ligand is an antibody, labelled antigen may be used as a receptor conjugate. Unbound receptor conjugate may thereafter be removed by a washing step. The presence of bound receptor conjugate on the solid phase system is then determined as an indication of the presence of the analyte in the sample.

This invention has been summarized in order that the drawings and detailed description that follow may be better understood and the contribution to the art may be better appreciated.

Figure 1 is a conceptualization of a porous matrix useful in the present invention and the manner of addition of microspheres thereto.

Figure 2 is a conceptualization of a cross section of a solid phase system of the present invention.

Figure 3 is a top view of a solid phase system of the present invention for the detection of a single analyte in a sample.

Figure 4 is a top view of a preferred solid phase system of the present invention for the multiple detection of different analytes in a sample.

Figure 5 is a top view of a preferred solid phase system of the present invention for the detection of a single analyte in a sample including internal positive and negative controls.

Figure 6 is a cross section of an apparatus incorporating a solid phase system of the present invention for performance of assays in accordance with the invention.

As indicated above, the present invention provides a solid phase system for use in ligand-receptor assays, particularly immunoassays, for the detection of a selected analyte in a fluid sample. In accordance with the present invention, the solid phase system comprises a porous matrix in which microspheres may be entrapped. A variety of porous matrices, including natural and synthetic matrices, may be suitably utilized provided microspheres of a size compatible with the pore size of the matrix may be entrapped in accordance with the invention. Among the matrices preferred for use are membranes or filters comprised of glass fibers, nylon, or ceramic materials having a defined porosity.

The solid phase system further comprises microspheres to which are bound a selected receptor, such as an antibody, preferably a monoclonal antibody, antigen or other suitable receptor substance capable of capturing the ligand of interest. Microspheres comprised of a polymeric material such/ as latex polyethylene, polypropylene,or polystyrene are preferred for use in the present invention. However, it will be recognised by those skilled in art that a variety of microspheres, comprised of either natural or synthetic materials, may be utilized. In the case of polymers, the polymer is selected on the basis of its ability to facilitate binding with the selected member of the ligand-receptor pair, e.g., it may be a polymer or copolymer having a functional group which facilitates binding by covalent bond formation or by a coating operation. Additionally, the microspheres are selected to have a size effective to permit their entrapment within the porous matrix as well as the flow of fluid around the microspheres and through the matrix. Preferred for use are microspheres having a size within the range of from about 0.1 to about 50 microns in diameter. We have found that microspheres having a size within this range tend to maximize the effective surface area available for reaction with the target ligand as aggregation and adhesion of the microspheres within the matrix is minimized. Particularly preferred for use are microspheres having a size within the range of from about 0.1 to about 1.0 microns in diameter; microspheres having a size within this range tend to enhance the contact and kinetics for reaction between receptor bound to the microspheres and the target ligand.

In accordance with the present invention, the microspheres selected for use are activated with a suitable receptor, e.g., an antibody, antigen or other biological substance suitable for use as a receptor to bind and capture the target ligand. Activation may be achieved by covalent binding or, in appropriate cases, by application of a coating of the receptor on the microspheres. In the case of an immunoassay to determine the presence of an antigen in a sample, the receptor is preferably a monoclonal antibody, however, polyclonal antibodies from antisera may be suitably used. Techniques for the coating or covalent binding of proteins to microspheres as well as for monoclonal and polyclonal antibody preparation, are well known to the art and require no repetition herein. The activated microspheres are entrapped in the matrix, preferably within a defined zone or zones of the matrix and in a predetermined pattern. Additionally, we have unexpectedly found that it is desirable to use an extremely low concentration of activated microspheres in solution for entrapment, preferably within the range of from about .01 to 1.0%, to provide increased sensitivity in an assay. The means for entrapment of the microspheres within the matrix is not critical and a variety of means may be used. For example, the microspheres may be added to the surface of the porous matrix as a suspension in a suitable liquid vehicle, for example, as a polymer latex, preferably a monodisperse latex, which is subsequently removed in a drying step. Gravity, a vacuum or capillary action may be used to draw the microspheres into discrete zones within the porous matrix prior to removal of the vehicle. As the microspheres are activated prior to entrapment, the present invention overcomes limitations associated with the preparation of the solid phase systems of the prior art.

In accordance with the present invention, the receptor-bound microspheres are preferably entrapped within the matrix in a manner which permits their mobility within the matrix and inhibits their agglutination or aggregation within the matrix, and further, which permits rapid fluid flow through the matrix and around the particles. While applicant does not intend to be bound by any theory, it is believed that the free movement of microspheres within the matrix may enhance the contact between receptor bound to the microspheres and the target ligand and permits effective washing during an assay process. Additionally, as indicated above, to attain the desired sensitivity of an assay process, the number and size of the entrapped microspheres must be optimized within the ranges set forth herein. Further, the microspheres may be coated prior to entrapment to minimize or inhibit their aggregation and adhesion within the matrix.

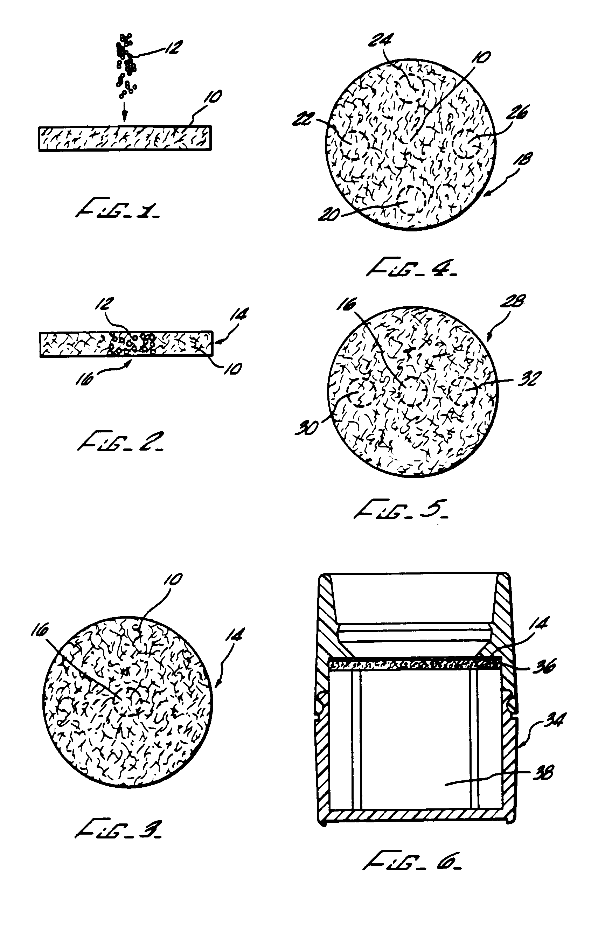

Turning now to Figure 1, there is shown in cross section a conceptualization of a porous matrix 10 useful in the present invention and the manner of addition of activated microspheres 12 thereto (microsphere vehicle is not shown). In Figure 2 there is shown in cross section a conceptualization of a solid phase system 14 of the present invention. Thus, in Figure 2, the porous matrix 10 is shown having the activated microspheres 12 entrapped in a defined or discrete zone 16 within the matrix 10. A top view of the solid phase system 14 for use in an assay for the detection of a single analyte, as conceptualized in Figure 2, is illustrated in Figure 3. Accordingly, in Figure 3, there is shown the discrete zone 16 in which microspheres 12 are entrapped for the capture of a ligand within the matrix 10 of the solid phase system 14.

A preferred solid phase system of the present invention comprises plural groups of microspheres entrapped in discrete zones, preferably in a predetermined pattern, within the matrix. Each group of microspheres is bound, prior to entrapment, with a different receptor, such as an antibody or antigen capable of capturing a different ligand of interest. Accordingly, in one embodiment of the invention, each group of microspheres comprises a population of microspheres bound with the same antibody, antigen or other receptor selected for use in the assay. Alternatively, a group of microspheres may comprise a mixture of microspheres to which are bound different receptors. For example, in the case of an immunoassay for an antigen, each group of microspheres may comprise at least two subpopulations of microspheres wherein each subpopulation is bound with an antibody, preferably a monoclonal antibody, capable of binding with a different determinant or epitope of the antigen. Preferably, the monoclonal antibodies bound with the subpopulations of microspheres comprising a distinct group of microspheres are selected to have a specific reactivity with non-interfering epitopes of the target ligand, thereby enhancing the sensitivity and specificity of the assay.

Accordingly, a solid phase system as described above is useful in a multiple ligand-receptor assay for the simultaneous detection of at least two selected analytes in a given sample. In the case of an immunoassay, the presence of different analytes within a sample may be detected simultaneously by a distinct color reaction for each analyte by the use of different enzyme labeled antibodies which generate distinct color reactions upon addition of a suitable substrate. Alternatively, different groups of microspheres may be entrapped within the porous matrix in a way which permits their identification by reason of their particular location in the matrix.

Further, the preferred solid phase system as described above is particularly useful where it is highly desirable to simultaneously determine the presence of more than one analyte of interest in a sample, such as for the determination of causative agents of an allergic response. This may be readily accomplished by entrapping distinct groups of microspheres, each of which is bound with one of a group of specific allergens (i.e., proteins or carbohydrates which are suspect in eliciting an immune response), within discrete zones of the matrix. Such a solid phase system provides, in essence, a panel of allergens capable of capturing IgE antibodies which may be present in a patient serum sample. Accordingly, the pattern of reactivity on the solid phase system, as determined by the presence of allergen-specific IgE antibodies in a given sample, provides an indication of the allergens eliciting a given allergic response. In such assays, typically the allergen-IgE pairs are detected by using labeled anti-IgE antibody as described in U.S. 3,720,760.

Referring now to Figure 4, there is shown a top view of a preferred solid phase system 18 for use in a multiple assay process in accordance with the conceptualization of a solid phase system 14 in Figure 2. Thus, in Figure 4 there is shown a matrix 10 in which there are discrete zones 20, 22, 24 and 26 comprising groups of microspheres, each of which is activated with a different receptor for the capture of different ligands of interest on the solid phase system 18.

Further in accordance with the present invention, a preferred solid phase system comprises distinct groups of microspheres as an internal control system (i.e., positive and/or negative controls). For example, the microspheres comprising a positive control may be bound with the target ligand or other suitable receptor substance capable of mimicing the binding of the target ligand. By comparison, the microspheres comprising a negative control are either without a bound component or, preferably, bound with a substance, e.g. antibody or antigen or DNA as the case may be, which is incapable of capturing the ligand of interest but which is capable of mimicing the non-specific binding interactions of the microsphere-bound receptor with components of the sample other than the target ligand. Such a solid phase system, which may be utiliized in a single or multiple assay, provides a means for determining the validity and reliability of the assay. Thus, in an immunoassay, the incorporation of a negative control provides a means for determining whether a false positive result, generally attributable to the nonspecific binding of a sample component with the receptor, has occurred. A positive control, on the other hand, reduces the likelihood that a false negative result will go undetected.

Additionally, the present invention provides a means for incorporating internal references for qualitative and quantitative determinations of a target ligand in an assay process as set forth and described in EP-A-0 253 464 assigned to the same assignee as the present invention.

Turning now to Figure 5, there is shown a top view of a preferred solid phase system 28 as described above for the detection of a single analyte and which incorporates internal controls. Thus, Figure 5 depicts, consistent with the conceptualization of Figure 2, a solid phase system 28 having a discrete zone 16 within the matrix 10 comprising microspheres to which the selected receptor is bound. Discrete zone 30 within the matrix 10 comprises microspheres to which the target ligand is bound as a positive control and discrete zone 32 within matrix 10 comprises microspheres which are preferably coated with a substance which mimics the non-specific binding characteristics of the receptor as a negative control. Thus, when system 28 is used in an assay of a patient sample which contains the analyte of interest, both zone 16 and 30 should indicate a positive result. On the other hand, a change in zone 32 corresponding with a change in zone 16 would be interpreted as a false positive result.

The apparatus of the present invention comprises, as a first porous member, a solid phase system as described above. The apparatus further comprises additional means, operatively associated with the first porous member, for facilitating the flow of a fluid sample and liquid reagents through the first member. Among the means which may be suitably utilized are means for applying a vacuum source or capillary action below the porous member to draw liquid through the porous member. Alternatively, the additional means may comprise means for applying a positive pressure above the porous member to force the sample liquid or reagents through that member. In a particularly preferred embodiment, a second absorbent member is associated with the solid phase system so as to permit the flow of a fluid sample through the solid phase system and into the absorbent material. The second absorbent member, having a surface over which the solid phase may be placed, has capillaries therethrough in a direction generally transverse to the surface over which the solid phase system is placed such that the capillaries are in direct communication with the pores of the solid phase system. A variety of materials may be used for the absobent member, Such as cellulose acetate fibers or polyester.

In an alternative embodiment the solid phase system may be placed on a porous support base which allows for the flow of a fluid sample through the solid phase system and into the absorbent material. The support base may consist of a porous disk, such as a polyethylene disk, and rest upon the absorbent member such that the absorbent member is in direct communication with the pores of the solid pahse system via the support base. The construction of such an apparatus may be, for example, essentially as described in US-A-4 632 901 and US-A-4 727 019.

An illustration of such an apparatus is provided by reference to Figure 6. Accordingly, in Figure 6, a solid phase system 14 such as the solid phase system depicted in top view by Figure 3 and conceptualized in cross section by Figure 2 is provided. The solid phase system 14 of the apparatus 34 rests on a porous support base 36 which permits the flow of a fluid sample through the solid phase system 14 and into an absorbent member 38 as described above.

As previously indicated, the solid phase system and apparatus of the present invention are of significant utility in the performance of ligand-receptor assays, particularly multiple ligand-receptor assays for the detection of at least two analytes of interest and/or ligand-receptor assays incorporating an internal control system. While the present invention is particularly useful for the performance of immunoassays, those skilled in the art will appreciate that with suitable modification, the solid phase system provided by the invention may be utilized for other ligand-receptor assays, including assays involving nucleic acid probe technology.

In accordance with the ligand-receptor processes of the invention, a fluid sample suspected of containing the analyte(s) of interest is introduced onto the solid phase system of an apparatus as described above. In the case of an immunoassay, the solid phase preferably incorporates as a receptor a monoclonal antibody bound to the microspheres, although a polyclonal antibody preparation may be used when the ligand of interest is an antigen. If the ligand of interest is an antibody, however, the solid phase may comprise an antigen for which the antibody to be detected is specific. Following the flow of fluid sample through the solid phase system and into the absorbent member, a solution of receptor conjugate, capable of binding with the target ligand and labeled so as to permit detection, is added. In the case of immunoassays, the receptor conjugate may be an antibody, preferably a monoclonal antibody, or antigen capable of binding with the ligand of interest. The addition of receptor conjugate, as appropriate, permits the formation of a complex with the ligand captured by the solid phase system. In the case of an immunoassay, if the microspheres comprising the solid phase system are bound with monoclonal antibody, and the labeled antibody is also a monoclonal antibody, the two antibodies are selected to bind to non-interfering binding sites of the antigen, essentially as described in U.S. 4,376,110 and U.S. 4,486,530, the disclosures of which are incorporated by reference. In presently preferred embodiments, the receptor conjugate is labeled with an enzyme, however other conventional labels, such as radionuclides, fluorescent agents, phosphorescent agents and polymers containing dyes or chemiluminescent moieties may be suitably utilized. After the solution of receptor conjugate has flowed through the solid phase system, a washing solution may be added to remove unbound receptor conjugate. Thereafter, the receptor conjugate complexed with the ligand of interest is detected. If an enzyme has been selected as the label component, the bound receptor conjugate is detected by the addition of an appropriate enzyme substrate to the solid phase system. Upon reaction with the substrate, the enzyme will generate, if properly selected, a color change on the solid phase system which may be detected by visual or instrumental means.

It will be recognized by those skilled in the art that assays for a broad range of analytes may be performed in accordance with the present invention. The list of potential analytes of interest is too lengthy for incorporation herein. However, antigens such as prostatic acid phosphatase, prostate-specific antigen, alphafetoprotein, carcinoembryonic antigen, leutenizing hormone, creatine kinase isoenzimes and other antigens in serum, plasma, urine or other biological fluids may be detected in an immunoassay. Additionally, the present invention is useful for the assay of viruses, bacteria, parasites or fungi, or antigens or antibodies associated therewith, including, for example, Rubella virus, Rota virus, adeno virus, respiratory syncitial virus, HTLV, hepatitis virus, hepatitis/A, hepatitis/B, hepatitis nonA nonB, influenza virus, cytomegalovirus and herpes virus, as well as group A and group B streptococcus, Neisseria gonorrhea, Trichomonas vaginalis**,** Candida albicans, Chlamydia trachomatis and Hemophilus influenza.

Further, it will be appreciated by those skilled in the art having the benefit of this disclosure that the present invention is applicable to assays involving nucleic acid probe technology. Specifically, a nucleic acid sequence complementary to a portion of the nucleic acid sequence of a ligand of interest may be bound to microspheres which are thereafter entrapped in a porous matrix. Such a solid phase system may be incorporated in an apparatus as previously described and utilized in assay processes. To perform such assay processes, a fluid sample suspected of containing the target ligand is introduced onto the solid phase and the ligand of interest is captured on the solid phase. Thereafter, a labeled nucleic acid probe having a nucleic acid sequence complementary to a different portion of the nucleic acid sequence of the target ligand is added to permit the formation of a complex of the labeled nucleic acid probe and the ligand captured on the solid phase. The detection of the labeled nucleic acid probe bound to the solid phase system provides an indication of the presence of the analyte in a given sample.

The following examples demonstrate the preparation of a solid phase system comprising microspheres entrapped within a glass fiber filter and the use of such a solid phase system to perform an immunoassay in accordance with the present invention. The examples are presented solely for purposes of illustration are not intended to limit the present invention in any way.

### EXAMPLE I

### DETECTION OF ANTIGEN

The assay set forth in the following example is an immunoassay for the detection of human choriogonadotripin (HCG), an antigen present in elevated levels in the urine of pregnant women.

### Activation of Microspheres

A monodisperse layer of polystyrene microspheres, 0.8 microns in diameter (Interfacial Dynamics Corporation, Portland, Oregon) were selected and activated by passive absorption of a preparation of monoclonal antibody derived from hybrid cell line HCU 061 (Hybritech Incorporated, San Diego, CA), specific for the HCG antigen. The microsphere size was selected to be compatible with Whatman GF/F glass fiber filter (Whatman Company, Clifton, New Jersey) by preliminary experimentation using fluorescent-labeled microspheres to determine retention within the filter. The monoclonal antibodies were obtained from ascites fluid and purified by Na₂SO₄ precipitation and DEAE ion exchange chromatography using standard methodology.

The antibody-bound microspheres were then backcoated with a 1% solution of Bovine Serum Albumin (BSA) in 50 mM phosphate (pH 8.0) to reduce non-specific binding of HCG and labeled antibody, following conventional procedures.

### Entrapment of Microspheres

A 10µl quantity of the activated microspheres were resuspended in 50 mM phosphate (pH 8.0) at 0.25% w/v concentration and pipetted onto the Whatman GF/F glass fiber filter incorporated in an immunoconcentration apparatus as shown in Figure 6. The microspheres were thereafter allowed to settle within the filter in a discrete circular zone at the center of the filter. The filter was allowed to air dry for 15 minutes.

### Detection of Antigen

Approximately 250µl of urine containing 125 mIU/ ml HCG were applied to the filter. Approximately 100/µl of a preparation of a second monoclonal antibody against HCG, derived from hybrid cell line HCO 514, and labeled with the enzyme alkaline phosphatase, was then added. After a 5 minute incubation during which time the enzyme-labeled antibody conjugate was drawn through the filter, the filter was washed with 2ml of 10mM Tris-buffered saline (pH 8.0). Approximately 100µl of a solution containing indoxyl phosphate, a substrate for the enzyme label was then added. After 5 minutes of incubation the filter was observed visually for a color reaction.

A dark blue color developed in the discrete zone of the filter where the HCU-061 activated microspheres were entrapped, indicating the presence of HCG antigen.

### EXAMPLE II

### ANTIBODY DETECTION

Antibodies to Rubella virus were detected in a manner similar to that described above for detection of HCG antigen in Example I. Microspheres of 0.8 microns in diameter which had been activated with purified Rubella antigen were entrapped in a discrete zone on the Whatman GF/F glass fiber filter incorporated in an immunoconcentration apparatus as shown in Figure 6. As a negative control, a group of non-antigen coated microspheres were entrapped in an adjacent zone on the filter.

### Detection of Antibodies

A 100 µl quantity of serum predetermined to be positive for the presence of antibodies to Rubella virus was applied onto a second filter prepared as described above. Simultaneously,a 100µl quantity of serum predetermined to be negative for the presence of anti-bodies on each filter with 2 ml of 10mM Tris-buffered saline (pH 8.0), 100/µl of enzyme conjugated-antibodies against human IgG (Kirkegaard & Perry Laboratories, Inc., Gaithersburg, Maryland) were added and allowed to react with the human IgG complexed with the Rubella antigen bound to the microspheres. After washing with 2 ml of 10 mM Tris-buffered saline (pH 8.0) to remove unbound conjugate, substrate for the enzyme label was added to detect the presence of any antigen-antibody complexes formed.

The positive sera resulted in a blue color in the discrete zone of the filter where microspheres bound with Rubella antigen were entrapped. (In the zone of the negative control, no color appeared.) The negative sera did not produce a color reaction.

It will be appreciated that the sensitivity of the assays of the examples may be adjusted by varying the volume and concentration of the microspheres, or incubation time, conjugate concentration, and other parameters commonly used in assay procedures.

## Claims

1. An apparatus for use in a ligand-receptor assay for the detection of at least one selected analyte in a fluid sample comprising:
(a) a first porous member comprising a solid phase system comprising:
(i) a porous matrix in which microspheres are entrapped wherein said microspheres are bound with a receptor capable of capturing a target ligand;
(ii) a porous matrix in which distinct groups of microspheres are entrapped within discrete zones wherein each distinct group of microspheres is bound with a receptor capable of capturing a different target ligand;
(iii) a porous matrix in which distinct groups of microspheres are entrapped within discrete zones, wherein at least one group of microspheres is bound with a receptor capable of capturing a target ligand, and at least one group of microspheres is bound with said target ligand or other suitable receptor substances as a positive control for the detection of said analyte; or
(iv) a porous matrix in which distinct groups of microspheres are entrapped within discrete zones, wherein at least one group of microspheres is bound with a receptor capable of capturing a target ligand, and at least one group of microspheres is bound with a substance incapable of capturing said target ligand, or without a bound component, as a negative control for the detection of said analyte; and
(b) a second absorbent member operatively associated with said first porous member, for facilitating the flow of said fluid sample and liquid reagent used in said assay through said first porous member.

2. An apparatus for use in a ligand-receptor assay for the detection of at least one selected analyte in a fluid sample comprising:
(a) a first porous member comprising a solid phase system comprising:
(i) a porous matrix in which microspheres are entrapped wherein said microspheres are bound with a receptor capable of capturing a target ligand;
(ii) a porous matrix in which distinct groups of microspheres are entrapped within discrete zones wherein each distinct group of microspheres is bound with a receptor capable of capturing a different target ligand;
(iii) a porous matrix in which distinct groups of microspheres are entrapped within discrete zones, wherein at least one group of microspheres is bound with a receptor capable of capturing a target ligand, and at least one group of microspheres is bound with said target ligand or other suitable receptor substances as a positive control for the detection of said analyte; or
(iv) a porous matrix in which distinct groups of microspheres are entrapped within discrete zones, wherein at least one group of microspheres is bound with a receptor capable of capturing a target ligand, and at least one group of microspheres is bound with a substance incapable of capturing said target ligand, or without a bound component, as a negative control for the detection of said analyte; and
(b) a second absorbent member associated with said first member so as to permit the flow of said fluid sample and liquid reagents used in said assay through said first member and into said second member, said second member having a surface over which said first member is placed and having capillaries therethrough in a direction generally transverse to the surface over which said first member is placed, which capillaries are in communication with the pores of said first member so as to draw fluid which has permeated said first member into the capillaries of said second member.

3. The apparatus according to claim 1 wherein said ligand-receptor assay is an immunoassay.

4. A ligand-receptor assay process for the detection of at least one selected analyte in a fluid sample comprising:
(a) introducing said fluid sample suspected of containing a target ligand onto the first porous member of the apparatus according to claim 1, which porous member comprises microspheres to which are bound a receptor capable of binding said target ligand;
(b) adding a solution of receptor conjugate capable of binding with said target ligand, said receptor conjugate being labelled to permit detection, in order to form a complex of said receptor conjugate with said target ligand;
(c) detecting said receptor conjugate, if any, bound to said porous member.

5. The process according to claim 4 wherein said ligand-receptor assay is an immunoassay.

6. The process according to claim 4 wherein said receptor is an allergen, said ligand is an IgE antibody specific for said allergen and said receptor conjugate is a labelled anti-IgE antibody.

7. The process according to claim 4 wherein said analyte is a virus or an antigen or antibody associated with said virus.

8. A process according to claim 7 wherein said ligand is selected from the group consisting of Rubella virus, Rota virus, adeno virus, respiratory syncitial virus, HTLV, hepatitis virus, hepatitis/A, hepatitis/B, hepatitis nonA nonB, influenza virus, cytomegalovirus or herpes virus.

9. The process according to claim 4 wherein said analyte is a bacterium, fungus or parasite or an antigen or antibody associated with said bacterium, fungus or parasite.

10. A process according to claim 9 wherein said analyte is selected from the group consisting of group A and B streptococcus, Neisseria aonorrhea, Trichomonas vaginalis, Candida albicans, Chlamydia trachomatis or Hemophilus influenza.

11. The process according to claim 4 wherein said analyte is a physiological marker substance selected from the group consisting of human choriogonadotropin, prostatic acid phosphatase, prostrate-specific antigen, alphafetoprotein, carcinoembryonic antigen, luteinizing hormone or creatine kinase isoenzyme.

12. The process according to claim 4 wherein said sample is serum, plasma, urine or other biological substance.

13. The process according to claim 4 wherein said receptor conjugate is labelled with an enzyme and said detecting step includes addition of a suitable substrate to said first porous member to generate a color change upon reaction with said enzyme which is determined by visual or instrumental means.

14. The process according to claim 4 wherein said receptor is a monoclonal antibody and said receptor conjugate is a labelled monoclonal antibody, said receptor and said receptor conjugate being selected to bind at non-interfering epitopes of said ligand.

15. The apparatus according to claim 1 wherein said ligand-receptor assay is a nucleic acid probe assay.

16. The process process according to claim 4 wherein said ligand-receptor assay is a nucleic acid probe assay.

17. The process according to claim 4 wherein said receptor is a nucleic acid sequence complementary to a portion of the nucleic acid sequence of said target ligand and said receptor conjugate is a labelled nucleic acid probe complementary to another portion of the nucleic acid sequence of said target ligand.

## Patentansprüche

1. Apparatur für die Verwendung in einem Ligand-Rezeptor-Assay für die Bestimmung mindestens eines ausgewählten Analyten in einer flüssigen Probe, umfassend: .
(a) einen ersten, ein Festphasensystem umfassenden Bestandteil, welcher umfaßt:
(i) eine poröse Matrix, in der Microsphären eingeschlossen sind, wobei die Microsphären an einen zur Bindung eines Zielliganden fähigen Rezeptor gebunden sind;
(ii) eine poröse Matrix, in der bestimmte Gruppen aus Microsphären in bestimmten Zonen eingeschlossen sind, wobei jede bestimmte Gruppe von Microsphären an einen zur Bindung eines anderen Zielliganden fähigen Rezeptor gebunden ist;
(iii) eine poröse Matrix, in der bestimmte Gruppen von Microsphären innerhalb bestimmter Zonen eingeschlossen sind, wobei mindestens eine Gruppe von Microsphären an einen zur Bindung eines Zielliganden fähigen Rezeptor gebunden ist und mindestens eine Gruppe von Microsphären an diesen Zielliganden oder andere geeignete Rezeptorsubstanzen als positive Kontrolle für die Bestimmung des Analyten gebunden ist; oder
(iv) eine poröse Matrix, in der bestimmte Gruppen von Microsphären innerhalb bestimmter Zonen eingeschlossen sind, wobei mindestens eine Gruppe von Microsphären an einen zur Bindung eines Zielliganden fähigen Rezeptor gebunden ist und mindestens eine Gruppe von Microsphären an eine nicht zur Bindung des Zielliganden fähigen Substanz gebunden ist, oder ohne gebundene Komponente, als negative Kontrolle für die Bestimmung des Analyten; und
(b) einen zweiten absorbierenden Bestandteil, der operativ mit dem ersten porösen Bestandteil verbunden ist, zur Erleichterung des Durchflusses der flüssigen Probe und des flüssigen Reagenz, die in dem Assay verwendet werden, durch den ersten porösen Bestandteil.

2. Apparatur für die Verwendung in einem Ligand-Rezeptor-Assay für die Bestimmung mindestens eines ausgewählten Analyten in einer flüssigen Probe, umfassend:
(a) einen ersten, ein Festphasensystem umfassenden Bestandteil, welcher umfaßt:
(i) eine poröse Matrix, in der Microsphären eingeschlossen sind, wobei die Microsphären an einen zur Bindung eines Zielliganden fähigen Rezeptor gebunden sind;
(ii) eine poröse Matrix, in der bestimmte Gruppen von Microsphären innerhalb bestimmter Zonen eingeschlossen sind, wobei jede bestimmte Gruppe von Microsphären an einen zur Bindung eines anderen Zielliganden fähigen Rezeptor gebunden ist;
(iii) eine poröse Matrix, in der bestimmte Gruppen von Microsphären innerhalb bestimmter Zonen eingeschlossen sind, wobei mindestens eine Gruppe von Microsphären an einen zur Bindung eines Zielliganden fähigen Rezeptor gebunden ist und mindestens eine Gruppe von Microsphären an diesen Zielliganden oder andere geeignete Rezeptorsubstanzen als positive Kontrolle für die Bestimmung des Analyten gebunden ist; oder
(iv) eine poröse Matrix, in der bestimmte Gruppen von Microsphären innerhalb bestimmter Zonen eingeschlossen sind, wobei mindestens eine Gruppe von Microsphären an einen zur Bindung eines Zielliganden fähigen Rezeptor gebunden ist und mindestens eine Gruppe von Microsphären an eine nicht zur Bindung des Zielliganden fähigen Substanz gebunden ist, oder ohne gebundene Komponente, als negative Kontrolle für die Bestimmung dieses Analyten; und
(b) einen zweiten absorbierenden Bestandteil, der mit dem ersten Bestandteil so verbunden ist, daß der Durchfluß der flüssigen Probe und der im Assay verwendeten flüssiger Reagenzien durch den ersten Bestandteil und in den zweiten Bestandteil ermöglicht wird, wobei der zweite Bestandteil eine Oberfläche aufweist, über der der erste Bestandteil plaziert wird, und durchgängige Kapillaren aufweist in einer Richtung, die im allgemeinen quer zu der Oberfläche verläuft, über der der erste Bestandteil plaziert wird, wobei die Kapillaren in Verbindung mit den Poren des ersten Bestandteils stehen, so daß Flüssigkeit, die den ersten Bestandteil durchdrungen hat, in die Kapillaren des zweiten Bestandteils gesogen wird.

3. Apparatur entsprechend Anspruch 1, wobei der Ligand-Rezeptor-Assay ein Immunoassay ist.

4. Ligand-Rezeptor-Assay-Verfahren für die Bestimmung mindestens eines ausgewählten Analyten in einer flüssigen Probe, umfassend:
(a) Einführen der flüssigen Probe mit dem vermuteten Zielliganden auf den ersten porösen Bestandteil der Apparatur entsprechend Anspruch 1, wobei der poröse Bestandteil Microsphären umfaßt, an die ein zur Bindung des Zielliganden fähiger Rezeptor gebunden ist;
(b) Zugabe einer Lösung von Rezeptorkonjugat, das mit dem Zielliganden binden kann, zur Bildung eines Komplexes der Rezeptorkonjugats mit dem Zielliganden, wobei das Rezeptorkonjugat zum Nachweis markiert ist;
(c) Nachweis des Rezeptorkonjugats, falls vorhanden, das an den porösen Bestandteil gebunden ist.

5. Verfahren entsprechend Anspruch 4, wobei der Ligand-Rezeptor-Assay ein Immunoassay ist.

6. Verfahren entsprechend Anspruch 4, wobei der Rezeptor ein Allergen ist, der Ligand ein für dieses Allergen spezifischer IgE-Antikörper ist und das Rezeptorkonjugat ein markierter Anti-IgE-Antikörper ist.

7. Verfahren entsprechend Anspruch 4, wobei der Analyt ein Virus oder ein mit diesem Virus assoziiertes Antigen oder ein Antikörper ist.

8. Verfahren entsprechend Anspruch 7, wobei der Ligand ausgewählt wird aus der aus Rubellavirus, Rotavirus, Adenovirus, Respiratory Syncitial Virus (RS-Virus), HTLV, Hepatitisvirus, Hepatitis/A, Hepatitis/B, Hepatitis nonA nonB, Influenzavirus, Cytomegalovirus oder Herpesvirus bestehenden Gruppe.

9. Verfahren entsprechend Anspruch 4, wobei der Analyt ein Bakterium, Pilz oder Parasit oder mit diesem Bakterium, Pilz oder Parasit assoziiertes Antigen oder ein Antikörper ist.

10. Verfahren entsprechend Anspruch 9, wobei der Analyt ausgewählt wird aus der aus Streptococcus Gruppe A und B, Neisseria gonorrhea, Trichomonas vaginalis, Candida albicans, Chlamydia trachomatis oder Hemophilus influenza bestehenden Gruppe.

11. Verfahren entsprechend Anspruch 4, wobei der Analyt eine physiologische Markierungssubstanz ist, ausgewählt aus der aus Humanchoriogonadotropin, Prostatinsäurephosphatase, Prostratspezifischem Antigen, Alphafetoprotein, Carcinoembryo-Antigen, luteinisierendem Hormon oder Kreatinkinase-Isoenzym bestehenden Gruppe.

12. Verfahren entsprechend Anspruch 4, wobei die Probe Serum, Plasma, Urin oder eine andere biologische Substanz ist.

13. Verfahren entsprechend Anspruch 4, wobei das Rezeptorkonjugat mit einem Enzym markiert wird und die Nachweisstufe Zugabe eines geeigneten Substrats zu dem ersten porösen Bestandteil zur Erzeugung eines Farbwechsels nach Reaktion mit dem Enzym beinhaltet, der durch visuelle oder instrumentelle Mittel bestimmt wird.

14. Verfahren entsprechend Anspruch 4, wobei der Rezeptor ein monoklonaler Antikörper ist, und das Rezeptorkonjugat ein markierter monoklonaler Antikörper ist, wobei der Rezeptor und das Rezeptorkonjugat so ausgewählt werden, daß sie an nicht interferierende Epitope des Liganden binden.

15. Apparatur entsprechend Anspruch 1, wobei der Ligand-Rezeptor-Assay ein Nukleinsäure-Sonden-Assay ist.

16. Verfahren entsprechend Anspruch 4, wobei der Ligand-Rezeptor-Assay ein Nukleinsäure-Sonden-Assay ist.

17. Verfahren entsprechend Anspruch 4, wobei der Rezeptor eine Nukleinsäuresequenz ist, die komplementär ist zur Nukleinsäuresequenz des Zielliganden, und wobei das Rezeptorkonjugat eine markierte Nukleinsäure-Sonde ist, die komplementär ist zur einem anderen Teil der Nukleinsäuresequenz des Zielliganden.

## Revendications

1. Dispositif pour utilisation dans un test de type ligand-récepteur pour la détection d'au moins un analyte choisi dans un échantillon fluide comprenant :
(a) un premier élément poreux comprenant un système en phase solide comprenant :
(i) une matrice poreuse dans laquelle sont piégées des microsphères où lesdites microsphères sont liées à un récepteur apte à capturer un ligand cible ;
(ii) une matrice poreuse dans laquelle des groupes distincts de microsphères sont piégés à l'intérieur de zones discrètes où chaque groupe distinct de microsphères est lié à un récepteur apte à capturer un ligand cible différent ;
(iii) une matrice poreuse dans laquelle des groupes distincts de microsphères sont piégés à l'intérieur de zones discrètes où au moins un groupe de microsphères est lié à un récepteur apte à capturer un ligand cible, et au moins un groupe de microsphères est lié audit ligand cible ou à d'autres substances récepteur appropriées en tant que témoin positif pour la détection dudit analyte ; ou
(iv) une matrice poreuse dans laquelle des groupes distincts de micro sphères sont piégés à l'intérieur de zones discrètes où au moins un groupe de microsphères est lié à un récepteur apte à capturer un ligand cible, et au moins un groupe de microsphères est lié à une substance incapable de capturer ledit ligand cible, ou bien est dépourvu de composé lié, en tant que témoin négatif pour la détection dudit analyte ; et
(b) un second élément absorbant associé de manière fonctionnelle audit premier élément poreux, pour faciliter l'écoulement dudit échantillon fluide et de réactif liquide utilisé dans ledit test à travers ledit premier élément poreux.

2. Dispositif pour utilisation dans un test de type ligand-récepteur pour la détection d'au moins un analyte choisi dans un échantillon fluide comprenant :
(a) un premier élément poreux comprenant un système en phase solide comprenant:
(i) une matrice poreuse dans laquelle sont piégées des microsphères où lesdites microsphères sont liées à un récepteur apte à capturer un ligand cible ;
(ii) une matrice poreuse dans laquelle des groupes distincts de microsphères sont piégés à l'intérieur de zones discrètes où chaque groupe distinct de microsphères est lié à un récepteur apte à capturer un ligand cible différent ;
(iii) une matrice poreuse dans laquelle des groupes distints de microsphères sont piégés à l'intérieur de zones discrètes où au moins un groupe de microsphères est lié à un récepteur apte à capturer un ligand cible, et au moins un groupe de microsphères est lié audit ligand cible ou à d'autres substances récepteur appropriées en tant que témoin positif pour la détection dudit analyte ; ou
(iv) une matrice poreuse dans laquelle des groupes distincts de micro sphères sont piégés à l'intérieur de zones discrètes où au moins un groupe de microsphères est lié à un récepteur apte à capturer un ligand cible, et au moins un groupe de microsphères est lié à une substance incapable de capturer ledit ligand cible ou bien est dépourvu de composé lié, en tant que témoin négatif pour la détection dudit analyte ; et
(b) un second élément absorbant associé audit premier élément de manière à permettre l'écoulement dudit échantillon fluide et des réactifs liquides utilisés dans ledit test à travers ledit premier élément et dans ledit second élément, ledit second élément ayant une surface sur laquelle est placé ledit premier élément et ayant des capillaires le traversant dans une direction généralement transversale à la surface sur laquelle ledit premier élément est placé, les capillaires étant en communication avec les pores dudit premier élément de manière à faire entrer du fluide qui est passé à travers ledit premier élément dans les capillaires dudit second élément.

3. Dispositif selon la revendication 1 dans lequel le test de type ligand-récepteur est un immunoessai.

4. Procédé de test de type ligand-récepteur pour la détection d'au moins un analyte choisi dans un échantillon fluide comprenant les étapes consistant à :
(a) introduire ledit échantillon fluide suspecté de contenir un ligand cible sur le premier élément poreux du dispositif selon la revendication 1, l'élément poreux comprenant des micro sphères auxquelles sont liées un récepteur apte à se lier audit ligand cible;
(b) ajouter une solution de conjugué de récepteur apte à se lier audit ligand cible, ledit conjugué de récepteur étant marqué pour permettre la détection, dans le but de former un complexe dudit conjugué de récepteur avec ledit ligand cible ;
(c) détecter ledit conjugué de récepteur, s'il est présent, lié audit élément poreux.

5. Procédé selon la revendication 4, dans lequel ledit test de type ligand-récepteur est un immunoessai.

6. Procédé selon la revendication 4, dans lequel ledit récepteur est un allergène, ledit ligand est un anticorps IgE spécifique dudit allergène et ledit conjugué de récepteur est un anticorps marqué anti-IgE.

7. Procédé selon la revendication 4, dans lequel ledit analyte est un virus ou un antigène ou anticorps associé audit virus.

8. Procédé selon la revendication 7, dans lequel ledit ligand est choisi dans le groupe constitué par le virus de la rubéole, un rotavirus, un adénovirus, le virus syncitial respiratoire, HTLV, un virus d'hépatite, hépatite/A, hépatite/B, hépatite non A non B, le virus de la grippe, le cytomégalovirus ou le virus de l'herpes.

9. Procédé selon la revendication 4, dans lequel ledit analyte est une bactérie, un champignon ou parasite ou un antigène ou anticorps associé à ladite bactérie ou audit champignon ou parasite.

10. Procédé selon la revendication 9, dans lequel ledit analyte est choisi dans le groupe constitué par les streptocoques du groupe A et du groupe B, Neisseria gonorrhea, Trichomonas vaginalis, Candida albicans, Chlamydia trachomatis ou Hemophilus influenza.

11. Procédé selon la revendication 4, dans lequel ledit analyte est un produit de marquage physiologique choisi dans le groupe constitué par la choriogonadotrophine humaine, la phosphatase acide prostatique, l'antigène spécifique de la prostate, l'alpha-fétoprotéine, l'antigène carcinoembryonnaire, l'hormone lutéinisante ou l'isoenzyme créatine kinase.

12. Procédé selon la revendication 4, dans lequel ledit échantillon est le sérum, le plasma, l'urine, ou une autre substance biologique.

13. Procédé selon la revendication 4, dans lequel ledit conjugué de récepteur est marqué avec une enzyme et ladite étape de détection comprend l'addition d'un substrat approprié audit premier élément poreux pour produire un changement de couleur par réaction avec ladite enzyme qui est déterminé par des moyens visuels ou de type instrumentaux.

14. Procédé selon la revendication 4, dans lequel ledit récepteur est un anticorps monoclonal et ledit conjugué de récepteur est un anticorps monoclonal marqué, ledit récepteur et ledit conjugué de récepteur étant choisis de manière à se lier à des épitopes dudit ligand non-interférents.

15. Dispositif selon la revendication 1, dans lequel ledit test de type ligand-récepteur est un test par sonde d'acide nucléique.

16. Procédé selon la revendication 4, dans lequel ledit test de type ligand-récepteur est un test par sonde d'acide nucléique.

17. Procédé selon la revendication 4, dans lequel ledit récepteur est une séquence d'acide nucléique complémentaire d'une partie de séquence d'acide nucléique dudit ligand cible et ledit conjugué de récepteur est une sonde d'acide nucléique marquée complémentaire d'une autre partie de séquence d'acide nucléique dudit ligand cible.
